(19) Europäisches Patentamt
European Patent Office
Office européen des brevets

(11) Publication number : **0 497 794 B1**

(12) # EUROPEAN PATENT SPECIFICATION

(45) Date of publication of patent specification : **13.07.94 Bulletin 94/28**

(51) Int. Cl.$^5$ : **C12N 9/08,** A61K 37/50

(21) Application number : **90915095.5**

(22) Date of filing : **12.10.90**

(86) International application number : **PCT/DK90/00260**

(87) International publication number : **WO 91/05858 02.05.91 Gazette 91/10**

(54) **A MICROPEROXIDASE PREPARATION CONTAINING A HEMOPEPTIDE AS AN ACTIVE COMPONENT.**

(30) Priority : **13.10.89 US 421414**

(43) Date of publication of application : **12.08.92 Bulletin 92/33**

(45) Publication of the grant of the patent : **13.07.94 Bulletin 94/28**

(84) Designated Contracting States : **AT BE CH DE DK ES FR GB GR IT LI LU NL SE**

(56) References cited :
**DE-A- 3 134 526**
**Chemical Abstracts, vol. 110. no. 7, 13 February 1989, (Columbus, Ohio, US), Adams, Paul A et al; p. 312, abstract 53642g**
**Chemical Abstracts, vol. 111, no. 19, 6 November 1989, (Columbus, Ohio, US), Adams, Paul A. et al; p. 328, abstract 169980r**
**Chemical Abstracts, vol. 105, no. 15, 13 October 1986, (Columbus, Ohio, US), Morgan, Robert O et al; p. 171, abstract 128340q**

(73) Proprietor : **NOVO NORDISK A/S**
**Novo Allé**
**DK-2880 Bagsvaerd (DK)**

(72) Inventor : **CHRISTENSEN, Bjoern, Eggert**
**Dronninggaards Allé**
**DK-2840 Holte (DK)**
Inventor : **SCHNEIDER, Palle**
**Rydtoften 43**
**DK-2750 Ballerup (DK)**
Inventor : **PEDERSEN, Gitte**
**Danasvej 6, 3.th**
**DK-1910 Frederiksberg C (DK)**

(74) Representative : **Knudsen, Sten Lottrup et al**
**c/o Novo Nordisk A/S,**
**Patent Department,**
**Novo Allé**
**DK-2880 Bagsvaerd (DK)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### TECHNICAL FIELD

This invention relates to a hemopeptide with peroxidase activity, to a peroxidase preparation, and to production and use of such a preparation.

### BACKGROUND ART

Peroxidase activity catalyses oxidation of a substrate (an electron or hydrogen donor such as lignin) with hydrogen peroxide.

High-molecular peroxidases (E.C. 1.11.1.7) are produced intracellularly by some microorganisms. Thus, S. Loprasert et al., *Journal of General Microbiology* (1988), **134**, 1971-1976) describe a peroxidase-catalase from *Bacillus stearo- thermophilus* with molecular weight 175,000, and US 4,698,306 describes a peroxidase from *Coprinus* with molecular weight 37,000-41,000. Also, so-called microperoxidases of mammalian origin are known; these are hemopeptides, typically with molecular weight in the range 1,500-3,000, exhibiting peroxidase activity (e.g. DE 3134526).

Use of peroxidase together with hydrogen peroxide or a hydrogen peroxide precursor has been suggested e.g. in bleaching of pulp for paper production (SE 88/0673), in treatment of waste water from pulp production (US 4,623,465, JP-A 2-31887) and for improved bleaching in laundry detergents (WO 89/09813). Pending US patent application S.N. 07/421,414 (filed 13 October 1989) and a co-pending PCT application disclose the use for dye transfer inhibition during laundering.

It is the object of the invention to provide an improved peroxidase for these purposes. The peroxidase should be active and stable at high temperature and $H_2O_2$ concentration, especially at alkaline conditions. It should be free of catalase as this activity breaks down hydrogen peroxide that is needed in the reaction. For better production economy, the peroxidase should be microbial and should be produced extracellularly by the microorganism in question.

### STATEMENT OF THE INVENTION

We have, surprisingly, discovered that peroxidase is produced extracellularly by some strains of *Bacillus pumilus.* The novel peroxidase preparation from *B. pumilus* is a microperoxidase preparation, i.e. it contains hemopeptide as an active component. The preparation has improved stability at high temperature, at high pH and at high concentrations of hydrogen peroxide. It can be produced without undesired catalase activity.

Accordingly, the invention provides a hemopeptide characterized by the formula:

```
Gln-Glu-Gln-Thr-Cys-Ile-Ser-Cys-His-Gly-Asp-Asn-Met-Gln
                 \         /
                   Heme
```

wherein the sulphur atoms of the two cysteine are linked to the vinyl groups of heme.

The invention also provides a peroxidase preparation, characterized by comprising as an active component a hemopeptide as defined above, and a peroxidase preparation, characterized by:

- comprising one or more active components derived from a strain of *B. pumilus,* with molecular weight in the range 800-2500, containing a heme group linked to a peptide chain containing the sequence Gln-Glu-Gln-Thr-Xaa-Ile-Ser-Yaa-His-Gly-Asp-Asn-Met-Gln, wherein Xaa and Yaa represent any amino acid,
- having a pH optimum in the range 7-9,
- retaining at least 40% residual activity after 30 minutes at 80°C and pH 7, and
- showing increasing reaction rate up to 20 mM $H_2O_2$.

The invention further provides a method of producing a peroxidase preparation, characterized by comprising cultivation of a peroxidase-producing strain of *B. pumilus* followed by recovery of peroxidase.

Finally, the invention provides use of the above peroxidase preparation together with hydrogen peroxide (optionally formed in situ) in bleaching of lignin-containing material.

## DETAILED DESCRIPTION OF THE INVENTION

Hemopeptide

The hemopeptide of the invention can be prepared synthetically by methods known in the art, viz. by first synthesizing the peptide chain including the two cysteines and then letting this peptide react with heme, or it can be produced genetically by methods known in the art: a degenerate oligonucleotide probe is synthesized (based on the known amino acid sequence) and used to isolate the *B. pumilus* gene encoding the precursor peptide chain of the microperoxidase. Modified microperoxidases are hereafter obtained by site directed mutagenesis (ref. Molecular Cloning, A Laboratory Manual, Cold Spring Harbor, 1989, Ed. J. Sambrook, E.F. Fritsh and T. Maniatis, ISBN 0-87969-309-6).

The following shows a comparison of the sequences of hemopeptides of the invention with a known microperoxidase (DE 3134526) prepared by hydrolysis of cytochrome C from animal or plant:

Prior art:

```
Phe-Val-Gln-Lys-Cys-Ala-Gln-Cys-His-Thr-Val-Glu-Lys-Gly
                  \        /
                     Heme
```

Invention:

```
Gln-Glu-Gln-Thr-Cys-Ile-Ser-Cys-His-Gly-Asp-Asn-Met-Gln
                  \        /
                     Heme
```

It is seen that the novel microbial microperoxidase has very little homology with the known microperoxidase.

Cultivation of *B. pumilus*

*B. pumilus* strains that can be used in the practice of the invention are atypical in their ability to produce peroxidase. Some *B. pumilus* strains, including the type strain, have been found not to produce peroxidase.

Two peroxidase-producing strains are freely available with deposit numbers ATCC 12905 and NCIB 8600, and one strain (internal designation S 197) has been deposited by the applicant under the terms of the Budapest Treaty; the deposit date was 23 July 1990, and the deposit number is DSM 6124.

ATCC indicates the American Type Culture Collection, 12301 Parklawn Drive, Rockville, Maryland 20852, U.S.A., NCIB indicates the National Collection of Industrial and Marine Bacteria Ltd., Torry Research Station, P.O. Box 31, 135 Abbey Road, Aberdeen AB9 8DG, Scotland, and DSM indicates Deutsche Sammlung von Mikroorganismen, Mascheroder Weg 1 B, 3300 Braunschweig, West Germany.

Peroxidase-producing *B. pumilus* strains can be cultivated under aerobic conditions in a nutrient medium containing assimilable carbon and nitrogen together with other essential nutrient. The medium can be composed in accordance with principles known in the art.

During cultivation, the cells secrete peroxidase extracellularly, while they apparently produce catalase intracellularly. Extracellular production of the peroxidase is advantageous as it allows for high fermentation yield and simple recovery. Catalase is undesired for most applications of peroxidase, so the recovery of peroxidase preferably includes separation of cell mass from the cell broth while avoiding cell lysis, e.g. by filtration or centrifugation.

The resulting cell-free culture broth contains a mixture of hemopeptides with peroxidase activity, mainly with molecular weight in the range 800-2500. This can be used as such, optionally after concentration e.g. by evaporation or ultrafiltration. If desired, the various hemopeptides can be separated and purified to the desired degree by conventional methods, e.g. by column chromatography.

pH and temperature dependence

A crude peroxidase preparation of the invention (fig. 1) and a similar purified preparation (fig. 2) have slightly different pH optimum, but both are in the range 7-9.

The thermostability of peroxidase preparations of the invention (fig. 3 and 4) is significantly better than a prior-art microbial peroxidase (fig. 5). A crude preparation according to the invention (fig. 3) is more thermostable than a similar purified preparation. Both preparations of the invention retain more than 40% residual activity after 30 minutes incubation at 80°C at pH 7.

Hydrogen peroxide dependence

A peroxidase preparation of the invention (fig. 4) shows increasing reaction rate up to 20 mM $H_2O_2$ (the highest tested). For comparison, a prior-art microbial peroxidase shows decreasing reaction rate at $H_2O_2$ concentrations above 0.1 mM (fig. 7).

Bleaching of lignin-containing material

Due to its good stability at high temperature and high $H_2O_2$ concentration, the peroxidase preparation of the invention is well suited for bleaching of lignin-containing materials, such as pulp for paper production or waste water from pulp manufacturing, together with hydrogen peroxide, e.g. as described in SE 88/0673, US 4,623,465 or JP-A 2-31887. Typical conditions are pH 7-10, 25-70°C, 10-300 mM $H_2O_2$ and a dosage of 10-100 NOPA/g dry matter. Hydrogen peroxide may be added as such or formed in situ, e.g. by incorporation of a precursor, such as a perborate or percarbonate, or of an enzymatic system capable of generating hydrogen peroxide, e.g. an oxidase and a substrate therefor (such as glucose and glucose oxidase).

Other uses of peroxidase

Use of the peroxidase preparation of the invention is particularly advantageous at high temperature and/or high $H_2O_2$ concentration and at alkaline pH. As an example, it can be incorporated into a laundry detergent together with a hydrogen peroxide precursor (such as sodium perborate or percarbonate) to improve the bleaching of stains according to WO 89/09813.

As another example, the peroxidase preparation can be used to inhibit the transfer of a textile dye from a dyed fabric to another fabric when the fabrics are washed and/or rinsed together in a wash liquor, by adding it together with a hydrogen peroxide precursor to the wash liquor in which the fabrics are washed and/or rinsed.

Peroxidase assay (NOPA)

Peroxidase activity is measured at 2 mM $H_2O_2$. The following are mixed in a 30°C thermostated 1 ml quartz cuvette:

200 µl 1 mM 4-aminoantipyrine (4-AA, Sigma No. A-4382, 0.2 mg/ml)
200 µl N-ethyl-N-sulphobutyl-m-toluidine-Na (ESBT, 5.86 mg/ml)
200 µl 0.5 M phosphate buffer, pH 7.0
200 µl enzyme sample, diluted to 0.02-0.10 NOPA/ml
200 µl 10 mM hydrogen peroxide is added, and the absorbance at 550 nm is followed for 1 minute. The activity is expressed in units denoted NOPA, calculated as the increase in absorbance within the first minute after addition of $H_2O_2$ multiplied by the dilution. The enzyme sample should be diluted so that the increase in absorbance per minute is within the limits 0.02 to 0.10.

## BRIEF DESCRIPTION OF DRAWINGS

Figures 1 and 2 show the pH-activity curve of a crude and a purified peroxidase preparation, respectively, according to the invention, measured by the NOPA method, but varying the pH as shown.

Figures 3 and 4 illustrate the thermostability of a crude and a purified peroxidase of the invention, respectively. For comparison, figure 5 shows the thermostability of a prior-art peroxidase preparation (from *Coprinus).* The measurements were made by incubating at the indicated time and temperature, then immediately diluting a sample 10 times in 0.1M phosphate buffer pH 7 at 25°C, and measuring peroxidase activity (NOPA).

Figures 6 and 7 illustrate reaction at various $H_2O_2$ concentrations with peroxidase of the invention and prior-art peroxidase *(Coprinus),* respectively. Oxidation of ESBT + 4-AA was followed by measuring the absorbance at 550 nm.

Figures 1 and 3 were made using cell-free culture broth prepared as in Example 1. Figures 2, 4, 6 and 7 were made using pool II from Example 1.

Figures 8 - 11 show chromatograms from purification of peroxidase of the invention. Details are given in Example 2.

## EXAMPLES

### EXAMPLE 1

Production of peroxidase from Bacillus pumilus

Media were prepared as follows (ingredients in g/l):

| | TY*3 |
|---|---|
| Trypticase, BBL g/l | 60 |
| Yeast Extract, Difco g/l | 15 |
| FeSO4*7H2O g/l | 0.025 |
| MnSO4*4H2O g/l | 0.0026 |
| MgSO4*7H2O g/l | 0.045 |
| pH | 7.3 (Adjusted with KOH) |

The medium was autoclaved at 121°C for 45 minutes

| | Agar3 |
|---|---|
| Peptone Bacto g/l | 6 |
| Pepticase g/l | 4 |
| Yeast Extract, Difco g/l | 3 |
| Meat Extract g/l | 1.5 |
| Glucose | 1 |
| pH | 7.3 |
| Agar (from Merck) | 20 (added last) |

The agar was autoclaved at 121°C for 45 minutes Inoculum agar : 10 Agar3 slants were inoculated with 1 freeze dried *Bacillus pumilus* Strain no S197 and incubated at 30°C for 24 hrs.

Inoculum media : Two 500 ml Shake flasks containing 100 ml TY∗3 media were inoculated with one Agar3 slant and incubated at 30°C and 250 rpm for 24 hrs.

Peroxidase production : 50 Shake flasks containing 100 ml of TY∗3 were inoculated each with 2 ml of inoculum described above. Then 2.5 ml of a sterile 40% (w/w) glucose in water was added to each shake flask. The shake flasks were incubated at 30°C for 48 hrs and then harvested. The final peroxidase activity was 1 NOPA/ml (NOPA measured at 20 mM $H_2O_2$).

**EXAMPLE 2**

Purification of peroxidase from *B. pumilus*

3250 ml of the culture broth obtained in Example 1 was filtered through a Seitz Supra 100 filter plate and secondly through a Supra 50 plate to obtain a clear filtrate with an activity of 1.29 NOPA/ml.

To 900 ml of the filtrate was added 70 g Amberlite XAD16 hydrophobic resin and the mixture was stirred overnight at 4°C. The resin was isolated by decantation and washed with 10%v/v ethanol. Peroxidase was extracted from the resin with 200 ml 50%v/v ethanol which was evaporated to 72 ml with an activity of 13.8 NOPA/ml (yield: 86%).

The evaporated peroxidase extract was applied to a DEAE Sepharose FF column (5x8 cm) equilibrated with 20 mM phosphate pH 7. The column was washed with more than one volume of buffer containing 0.1 M NaCl and peroxidase was eluted with buffer containing 0.3 M NaCl. Fractions of 10 ml were collected and pooled in three pools according to protein (A280) and activity profiles (fig. 8):

| | Fraction no. | ml | A280 | A404 | NOPA/ml | Yield |
|---|---|---|---|---|---|---|
| Pool A | 23-26 | 40 | 8.14 | 1.37 | 2.33 | 9.3% |
| Pool B | 27-30 | 40 | 6.41 | 1.05 | 2.17 | 8.7% |
| Pool C | 36-42 | 40 | 8.90 | 1.84 | 6.89 | 27.7% |

A 90 ml sample with 7.34 NOPA/ml of pool C was added 1.6 M ammonium sulphate to a conductivity of 108 mS (pH 7.05) and applied on an Octyl-Sepharose column (5x11 cm) equilibrated with 0.8 M ammonium sulphate pH 7. Unbound material was eluted with 0.8 M ammonium sulphate and peroxidase activity was then eluted with a linear gradient of ammonium sulphate from 0.8 to 0 molar (pure water).

Fractions of 10 ml were collected and pooled in three pools according to the peroxidase activity (fig 9):

| | Fraction no. | ml | A280 | A398 | NOPA/ml | Yield |
|---|---|---|---|---|---|---|
| Pool I | 84-92 | 90 | 0.26 | 0.19 | 1.47 | 20.0% |
| Pool II | 94-96 | 30 | 0.58 | 0.37 | 2.09 | 9.5% |
| Pool III | 97-100 | 40 | 0.55 | 0.30 | 1.57 | 9.5% |

A sample of pool I was concentrated by ultrafiltration on a 50 ml Amicon stirred cell with a DDS GS90PP membrane. A 7.5 ml concentrated sample with an activity of 131.2 NOPA/ml was applied to a Sephacryl-S200 column (2.5x85 cm) equilibrated with 12.5 mM phosphate buffer pH 7.0 and eluted with the same buffer at a flow rate of 0.9 ml/min. Fractions of 9.8 ml were collected and pooled according to peroxidase activity and spectral properties (Rz-value: A398/A280) (fig. 10):

| | Fraction no. | ml | A280 | A398 | NOPA/ml | Yield |
|---|---|---|---|---|---|---|
| Pool IA | 47-50 | 38 | 0.47 | 1.22 | 11.2 | 43.3% |
| Pool IB | 45 | 9.8 | 0.73 | 0.69 | 6.76 | 6.7% |
| Pool IC | 46 | 9.8 | 1.16 | 1.65 | 15.97 | 15.8% |

The mass spectrum of Pool IA show one major peak at Mw 2214.0/2228.1 and two minor peaks at Mw

972.8 and 1768.1, respectively. The amino acid sequence of the major component was shown to be:

Gln-Glu-Gln-Thr-?-Ile-Ser-?-His-Gly-Asp-Asn-Met-Gln

where the two missing amino acids both are believed to be cysteine through which a heme group (responsible for the A398 absorbance) can be bound.

A sample of pool II was concentrated by ultrafiltration on a 50 ml Amicon stirred cell with a DDS GS90PP membrane. A 10 ml concentrated sample with an activity of 143.8 NOPA/ml was applied to a AcA 202 column (2.5x85 cm) equilibrated with 12.5 mM phosphate buffer pH 7.0 and eluted with the same buffer at a flow rate of 0.4 ml/min. Fractions of 5 ml were collected and monitored for A280, A398 and peroxidase activity (fig. 11).

This AcA 202 gel is claimed by the manufacturer to separate molecules in the range from 1 kD to 15 kD. From the chromatogram it is seen that a wide range of relatively low Mw substance was eluted and all showing the same peroxidase activity relative to the A398 (Soret) absorbance.

**EXAMPLE 3**

Bleaching of kraft pulp

The peroxidase used in this example was the permeate from ultrafiltration of the supernatant from alcohol precipitation of a cell-free culture broth (obtained essentially as in Example 1).

1 g hardwood (birch) kraft pulp, delignified by oxygen, was blended in 250 ml water adjusted to pH 8.0-8.2 with NaOH. $H_2O_2$ was added to a concentration of 1% (0.3M), and the above peroxidase preparation was added to a dosage of 0.32 NOPA/ml. The mixture was incubated at 40°C for 2 hours. Then the pulp was collected on a Buchner funnel and washed with sufficient water to obtain a clear filtrate. The pulp was pressed and dried, and the ISO brightness of the obtained sheet was measured. A reference was made in the same way without the enzyme.

Invention: 57.2% ISO brightness
Reference: 55.6% ISO brightness

**EXAMPLE 4**

Bleaching of CTMP pulp

The experiment of Example 3 was repeated at 30°C with CTMP softwood (pine) pulp.

Invention: 59.7% ISO brightness
Reference: 56.1% ISO brightness

**EXAMPLE 5**

Bleaching of dyes in solution

The effect of the peroxidase preparation of the invention in bleaching a dissolved textile dye was tested. The dyestuffs tested were Direct Blue 1 (C.I. #24410, product of Keystone Aniline), Acid Red 151 (C.I. #26900, product of Sandoz), Procion Blue H ERD (product of ICI) and Procion Blue H EXL (product of ICI). The peroxidase preparation was cell-free culture broth prepared essentially as in Example 1.

A reaction solution was prepared, containing 50 mM sodium phosphate, 0.3 NOPA/ml of peroxidase, dyestuff (as indicated below) corresponding to an absorption maximum (in the visible range) of 0.025-0.035, and 0.25 mM $H_2O_2$ at room temperature at the pH indicated below. After addition of $H_2O_2$ (added last), a spectral scan was made every minute for 12 minutes. Below, the change in absorbance at the wavelength of maximum absorption is listed.

| Dyestuff | pH | Absorbance change immediately/after 12 min | Wave-length |
|---|---|---|---|
| Acid Red 151 | 7.0 | 0.030/0.032 | 513 nm |
| | 9.0 | 0.033/0.033 | 513 - |
| | 10.5 | 0.027/0.030 | 513 - |
| Direct Blue 1 | 7.0 | 0.024/0.025 | 597 nm |
| | 9.0 | 0.022/0.026 | 597 - |
| | 10.5 | 0.009/0.023 | 597 - |
| Procion Blue H ERD | 7.0 | 0.022/0.021 | 617 nm |
| | 9.0 | 0.009/0.022 | 617 - |
| | 10.5 | 0.001/0.010 | 617 - |
| Procion Blue H EXL | 9.0 | 0.021/0.026 | 626 nm |
| | 10.5 | 0.016/0.025 | 626 - |

When the two values of the absorbance change are close, the bleaching is practically instantaneous. Generally, bleaching over the entire visible range follows the above trends at the absorbance maximum.

In all cases, use of 0.25 mM $H_2O_2$ without enzyme left the dye unchanged.

This example illustrates the potential usefulness of the peroxidase preparation of the invention in preventing surplus dye from strongly-coloured fabric from being redeposited during washing.

## Claims

1. A hemopeptide characterized by the formula:

```
Gln-Glu-Gln-Thr-Cys-Ile-Ser-Cys-His-Gly-Asp-Asn-Met-Gln
                  \         /
                     Heme
```

   wherein the sulphur atoms of the two cysteine are linked to the vinyl groups of heme.

2. A peroxidase preparation, characterized by comprising the hemopeptide according to Claim 1 as an active component.

3. A peroxidase preparation, characterized by:

- comprising one or more active components derived from a strain of *B. pumilus*, with molecular weight in the range 800-2500, containing a heme group linked to a peptide chain containing the sequence Gln-Glu-Gln-Thr-Xaa-Ile-Ser-Yaa-His-Gly-Asp-Asn-Met-Gln, wherein Xaa and Yaa represent any amino acid,
- having a pH optimum in the range 7-9,
- retaining at least 40% residual activity after 30 minutes at 80°C and pH 7, and
- showing increasing reaction rate up to 20 mM $H_2O_2$.

**4.** A peroxidase preparation according to Claim 3, wherein the strain of *B. pumilus* strain is DSM 6124, ATCC 12905 or NCIB 8600.

**5.** A catalase-free peroxidase preparation according to any of Claims 2-4.

**6.** A method of producing a peroxidase preparation, characterized by comprising cultivation of a peroxidase-producing strain of *B. pumilus,* followed by recovery of peroxidase.

**7.** A method according to Claim 6, wherein the strain of *B. pumilus* produces peroxidase extracellularly, and the recovery comprises removal of cell mass.

**8.** A method according to Claim 11 or 12, wherein the strain of *B. pumilus* is DSM 6124, ATCC 12905 or NCIB 8600.

**9.** Use of the peroxidase preparation of any of Claims 2 - 5 or of a preparation produced according to any of Claims 6 - 8 together with hydrogen peroxide (optionally formed in situ) in bleaching of lignin-containing material.

**10.** Use according to Claim 9 in bleaching of pulp for paper production or waste water from pulp manufacturing.

## Patentansprüche

**1.** Ein Hämopeptid, gekennzeichnet durch die Formel:

Gln-Glu-Gln-Thr-Cys-Ile-Ser-Cys-His-Gly-Asp-Asn-Met-Gln
\ /

Häm

wobei die Schwefelatome der zwei Cysteine an die Vinylgruppen von Häm gebunden sind.

**2.** Ein Peroxidasepräparat, dadurch gekennzeichnet, daß es das Hämopeptid nach Anspruch 1 als eine aktive Komponente umfaßt.

**3.** Ein Peroxidasepräparat, dadurch gekennzeichnet, daß es:

- eine oder mehrere aktive Komponenten umfaßt, gewonnen aus einem Stamm von B. pumilus, mit einer relativen Molekülmasse im Bereich von 800-2.500, die eine Häm-Gruppe enthält (enthalten), die an eine Peptidkette gebunden ist, die die Sequenz Gln-Glu-Gln-Thr-Xaa-Ile-Ser-Yaa-His-Gly-Asp-Asn-Met-Gln enthält, wobei Xaa und Yaa irgendeine Aminosäure repräsentieren,
- ein pH-Optimum im Bereich 7-9 besitzt,
- wenigstens 40 % Restaktivität nach 30 Minuten bei 80°C und pH 7 zurückbehält und
- ansteigende Reaktionsgeschwindigkeit bis zu 20 mM $H_2O_2$ zeigt.

**4.** Ein Peroxidasepräparat nach Anspruch 3, wobei der Stamm von B. pumilus-Stamm DSM 6124, ATCC 12905 oder NCIB 8600 ist.

**5.** Ein katalasefreies Peroxidasepräparat nach einem der Ansprüche 2-4.

**6.** Ein Verfahren zur Herstellung eines Peroxidasepräparates, dadurch gekennzeichnet, daß es die Kultivierung eines Peroxidase-produzierenden Stammes von B. pumilus umfaßt, gefolgt von der Gewinnung von Peroxidase.

**7.** Ein Verfahren nach Anspruch 6, wobei der Stamm von B. pumilus Peroxidase extrazellulär produziert und die Gewinnung das Entfernen von Zellmasse umfaßt.

**8.** Ein Verfahren nach Anspruch 11 oder 12, wobei der Stamm von B. pumilus DSM 6124, ATCC 12905 oder NCIB 8600 ist.

**9.** Verwendung des Peroxidasepräparats nach einem der Ansprüche 2-5 oder eines nach einem der Ansprüche 6-8 hergestellten Präparats zusammen mit Wasserstoffperoxid (fakultativ in situ gebildet) beim Bleichen von ligninhaltigem Material.

**10.** Verwendung nach Anspruch 9 beim Bleichen von Zellstoff zur Papierherstellung oder Abwasser aus der Zellstoffherstellung.

## Revendications

**1.** Hémopeptide caractérisé par la formule :

```
Gln-Glu-Gln-Thr-Cys-Ile-Ser-Cys-His-Gly-Asp-Asn-Met-Gln
                  \        /
                     Heme
```

dans laquelle les atomes soufre des deux cystéines sont liés aux groupes vinyle d'hème.

**2.** Préparation de peroxydase, caractérisée en ce qu'elle comprend l'hémopeptide selon la revendication 1, comme composant actif.

**3.** Préparation de peroxydase, caractérisée par le fait de :

- comprendre un ou plusieurs composants actifs dérivés d'une souche de B. pumilus, d'une masse moléculaire dans la plage de 800-2500, contenant un groupe hème lié à une chaîne peptide contenant la séquence Gln-Glu-Gln-Thr-Xaa-Ile-Ser-Yaa-His-Gly-Asp-Asn-Met-Gln, dans laquelle Xaa et Yaa représentent tout acide aminé.
- posséder un pH optimum dans la plage de 7-9,
- conserver au moins 40 % d'activité résiduelle après 30 minutes à 80°C et pH 7, et
- présenter un taux de réaction croissant jusqu'à 20 mM $H_2O_2$.

**4.** Préparation de peroxydase selon la revendication 3, dans laquelle la souche de B. pumilus est DSM 6124, ATCC 12905 ou NCIB 8600.

**5.** Préparation de peroxydase exempte de catalase selon l'une quelconque des revendications 2-4.

**6.** Procédé de production d'une préparation de peroxydase, caractérisé par le fait de comprendre la mise en culture d'une souche productrice de peroxydase de B. pumilus, suivie de la récupération de la peroxydase.

**7.** Procédé selon la revendication 6, dans lequel la souche de B. pumilus produit de la peroxydase extracellulairement et la récupération comprend l'extraction de la masse cellulaire.

**8.** Procédé selon la revendication 11 ou 12, dans lequel la souche de B. pumilus est le DSM 6124, ATCC 12905 ou NCIB 8600.

**9.** Utilisation de la préparation de peroxydase selon l'une quelconque des revendications 2-5 ou d'une préparation produite selon l'une quelconque des revendications 6-8 conjointement avec le peroxyde d'hydrogène (facultativement formé in situ) pour le blanchiment de matières contenant de la lignine.

**10.** Utilisation selon la revendication 9, dans le blanchiment de pâte à papier pour la production de papier ou d'eaux usées provenant de la fabrication de pâte à papier.

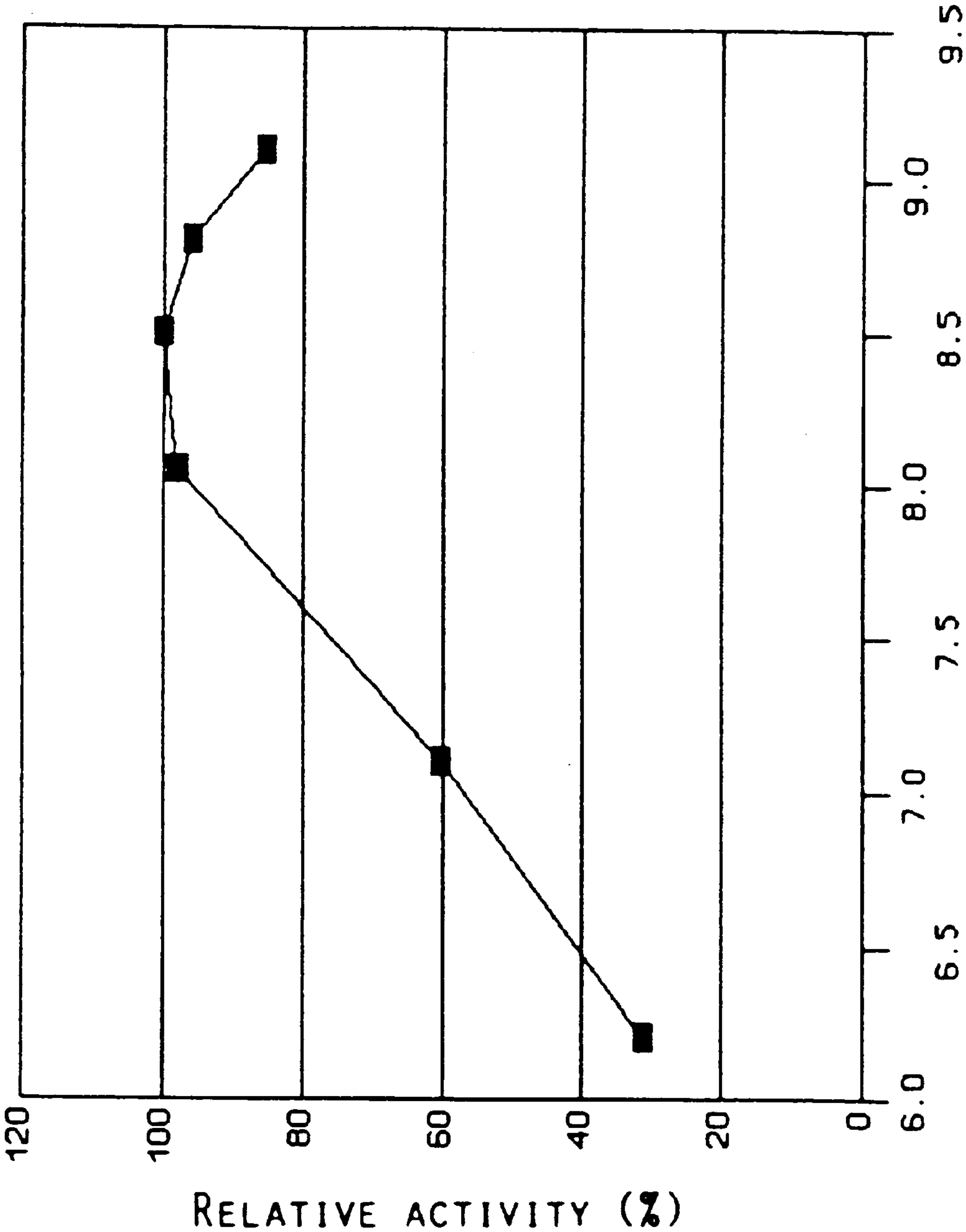


Fig. 1

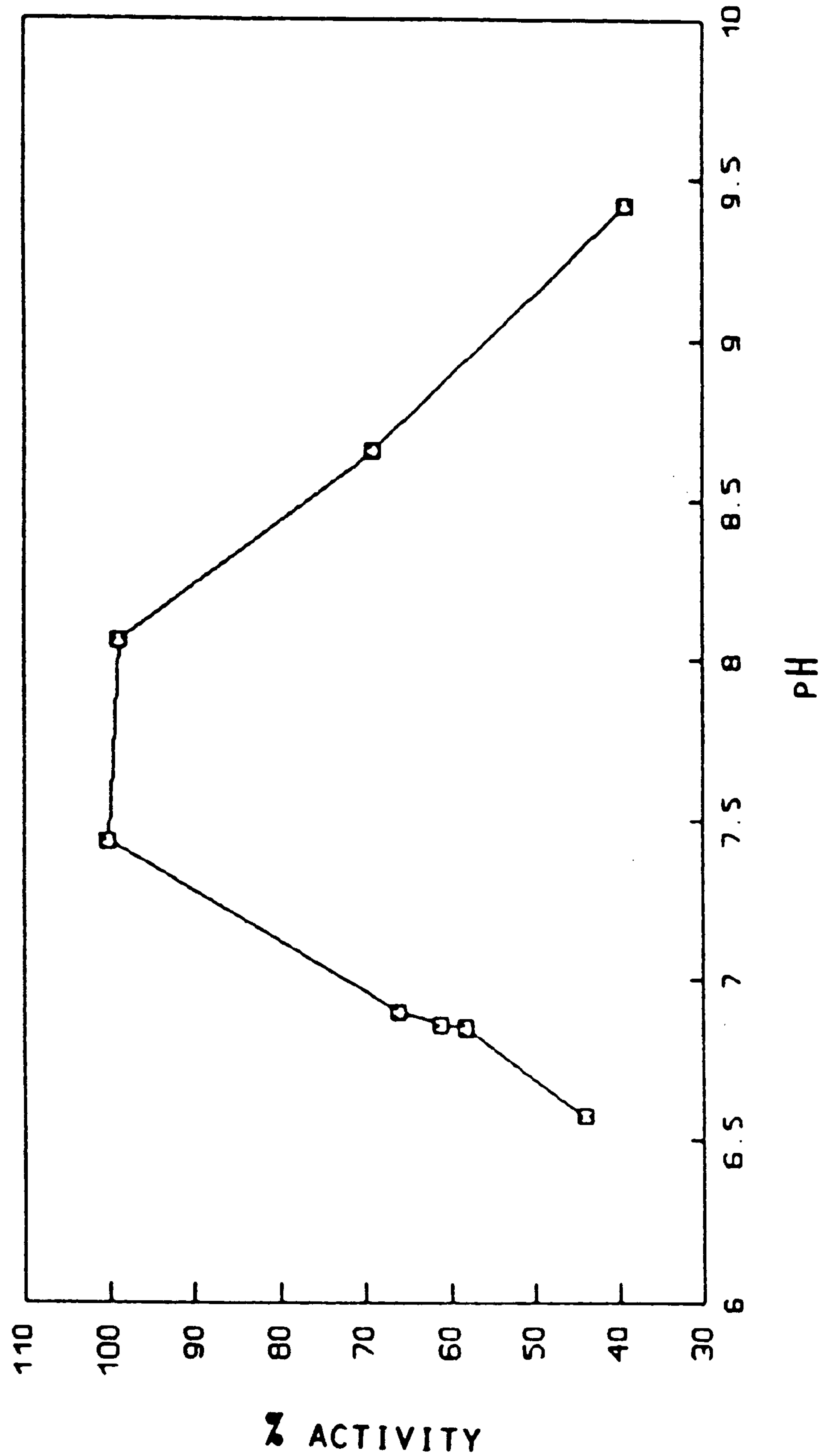


Fig. 2

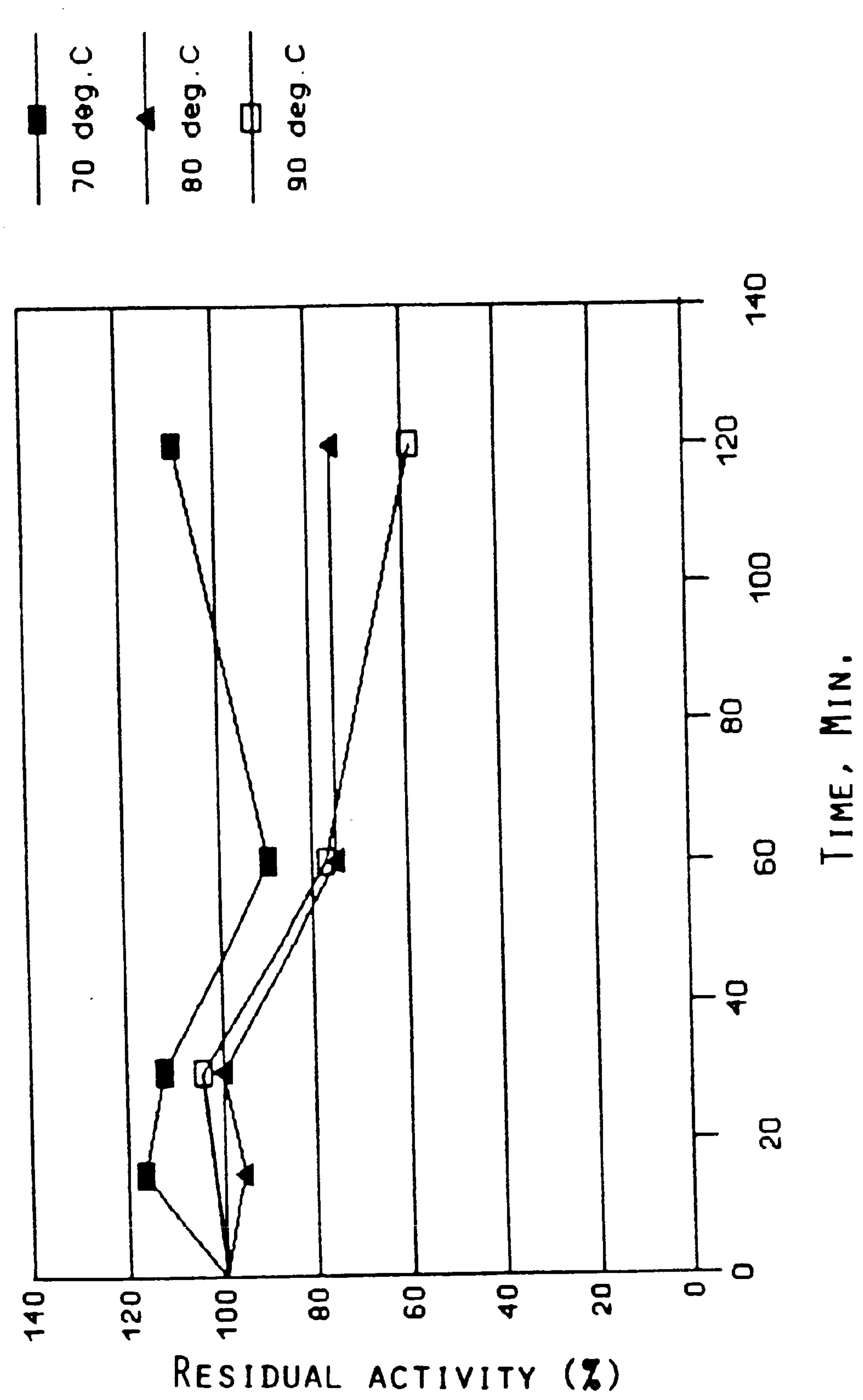
70 deg.C
80 deg.C
90 deg.C
RESIDUAL ACTIVITY (%)
140
120
100
80
60
40
20
0
TIME, MIN.
0
20
40
60
80
100
120
140

Fig. 3

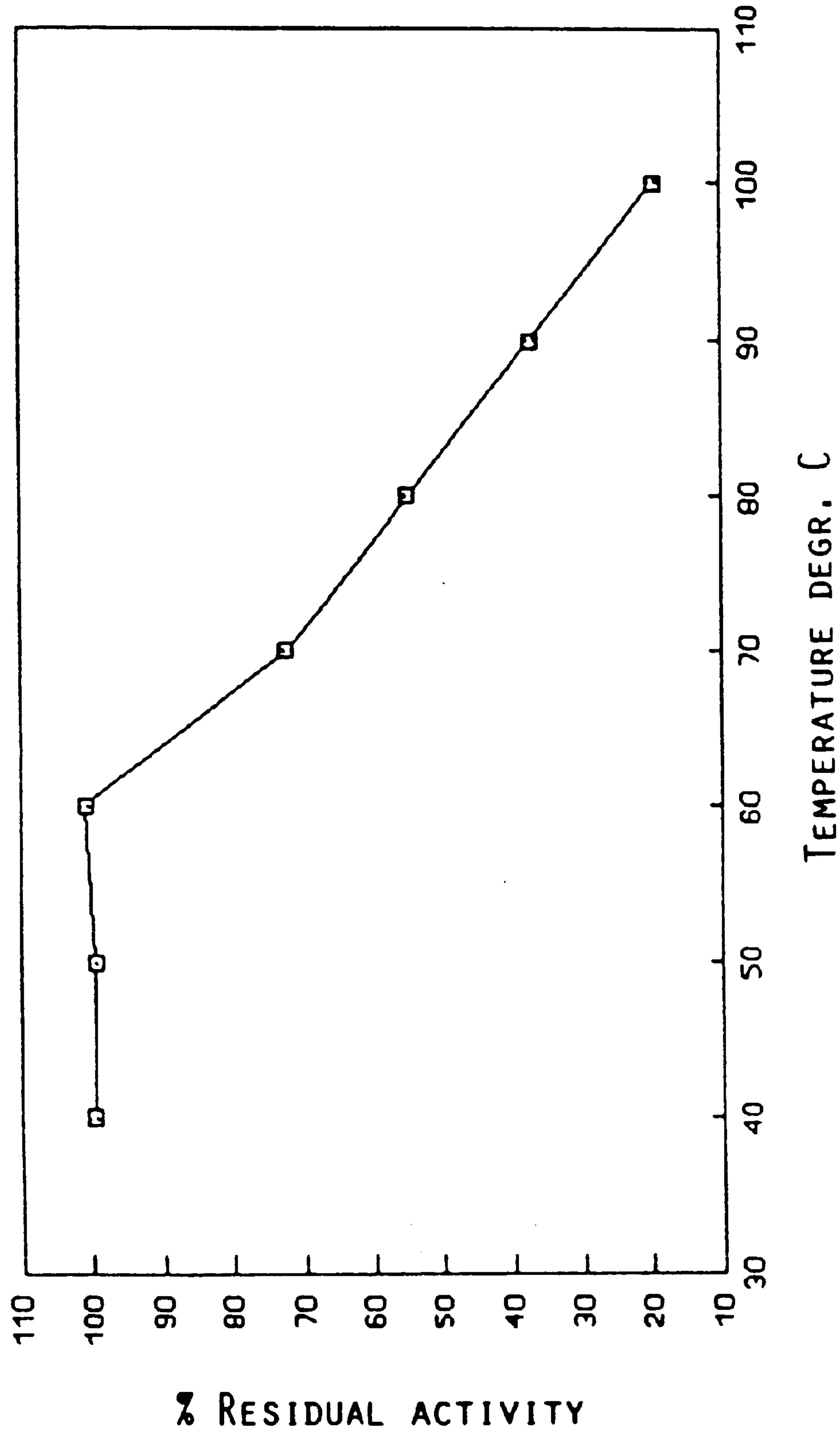
% RESIDUAL ACTIVITY
110
100
90
80
70
60
50
40
30
20
10
TEMPERATURE DEGR. C
30
40
50
60
70
80
90
100
110

Fig. 4

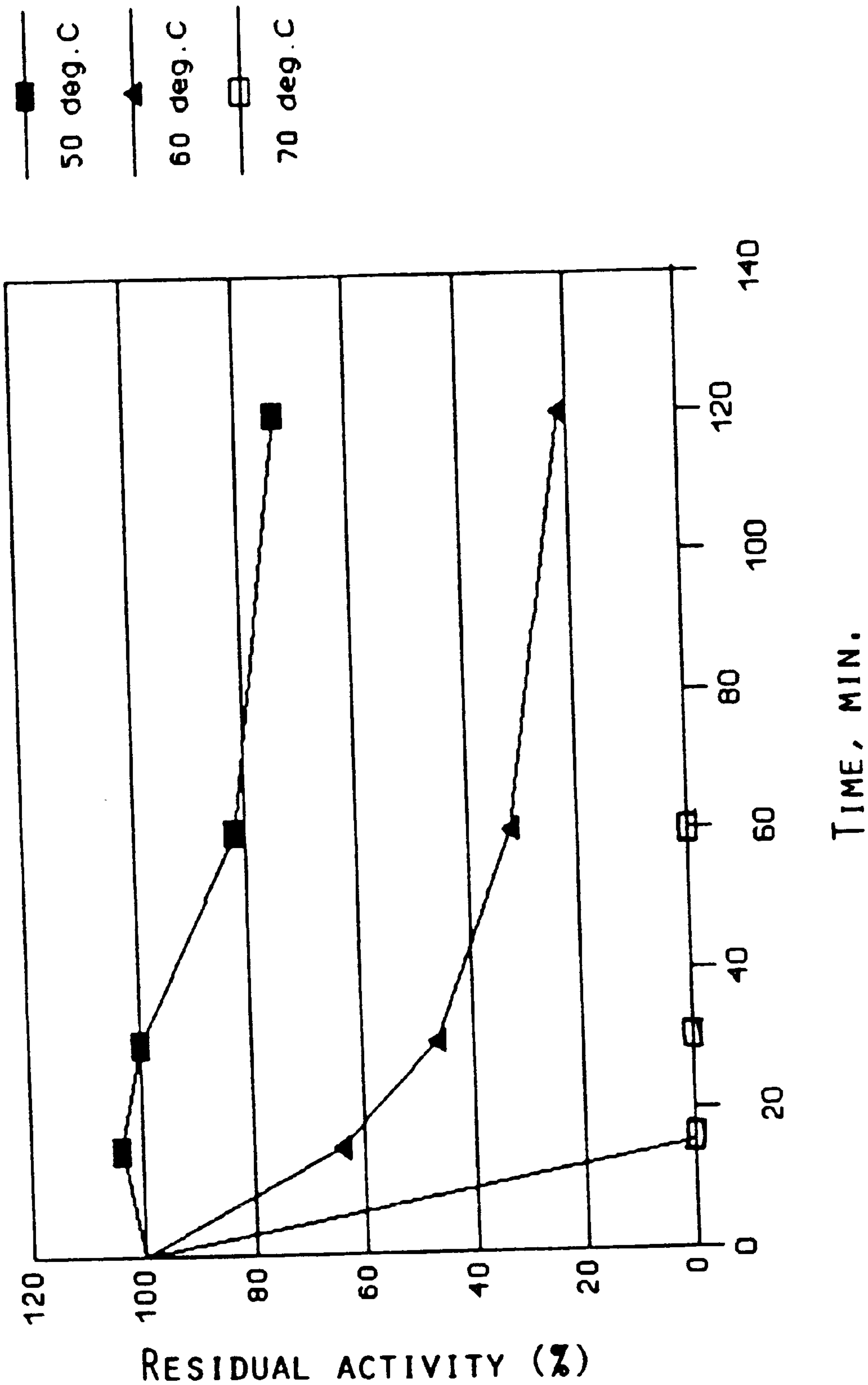
50 deg.C
60 deg.C
70 deg.C
RESIDUAL ACTIVITY (%)
0
20
40
60
80
100
120
TIME, MIN.
0
20
40
60
80
100
120
140

Fig. 5

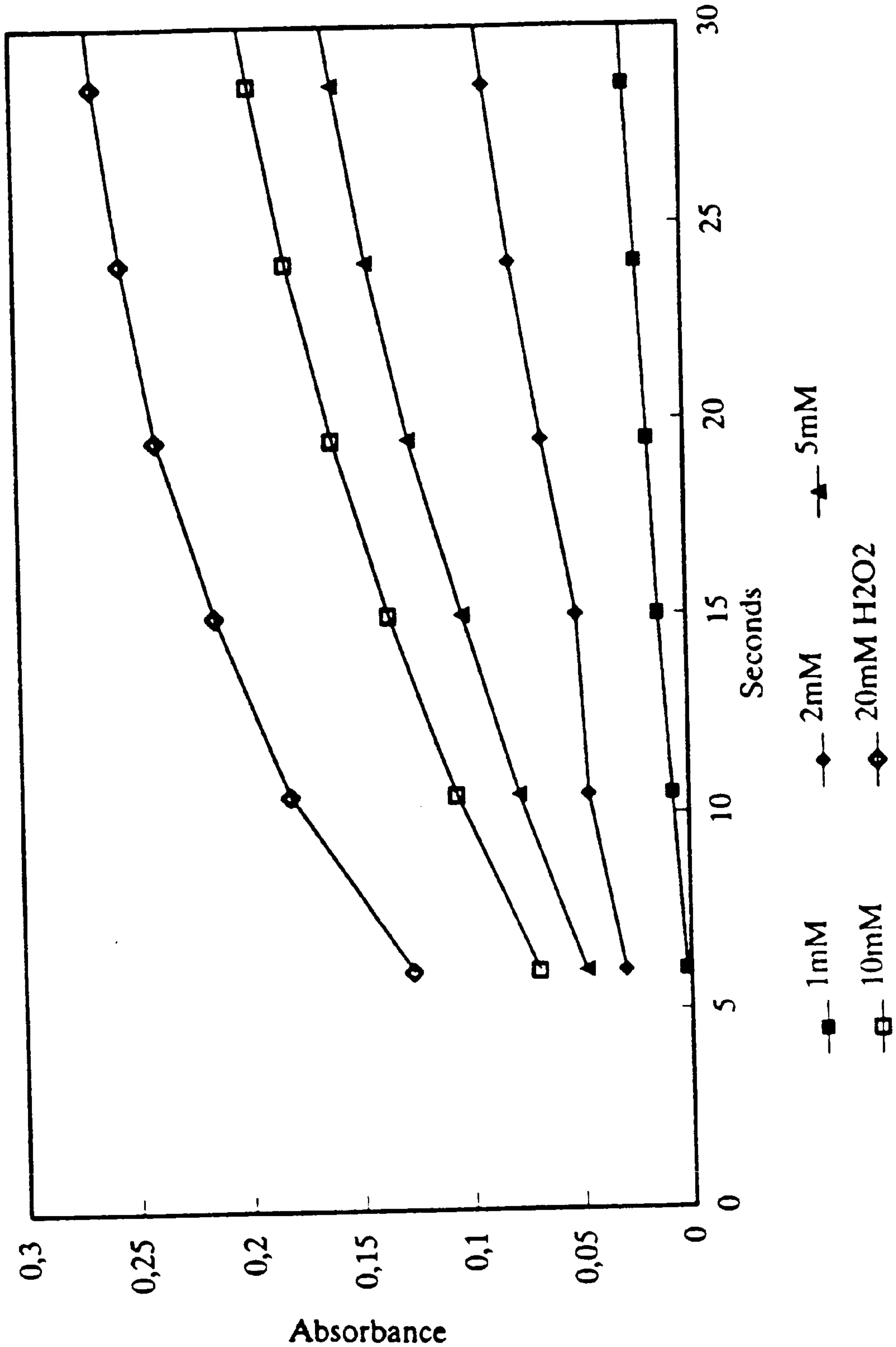

Absorbance
0,3
0,25
0,2
0,15
0,1
0,05
0
0
5
10
15
20
25
30
Seconds
1mM
2mM
5mM
10mM
20mM H2O2


Fig. 6

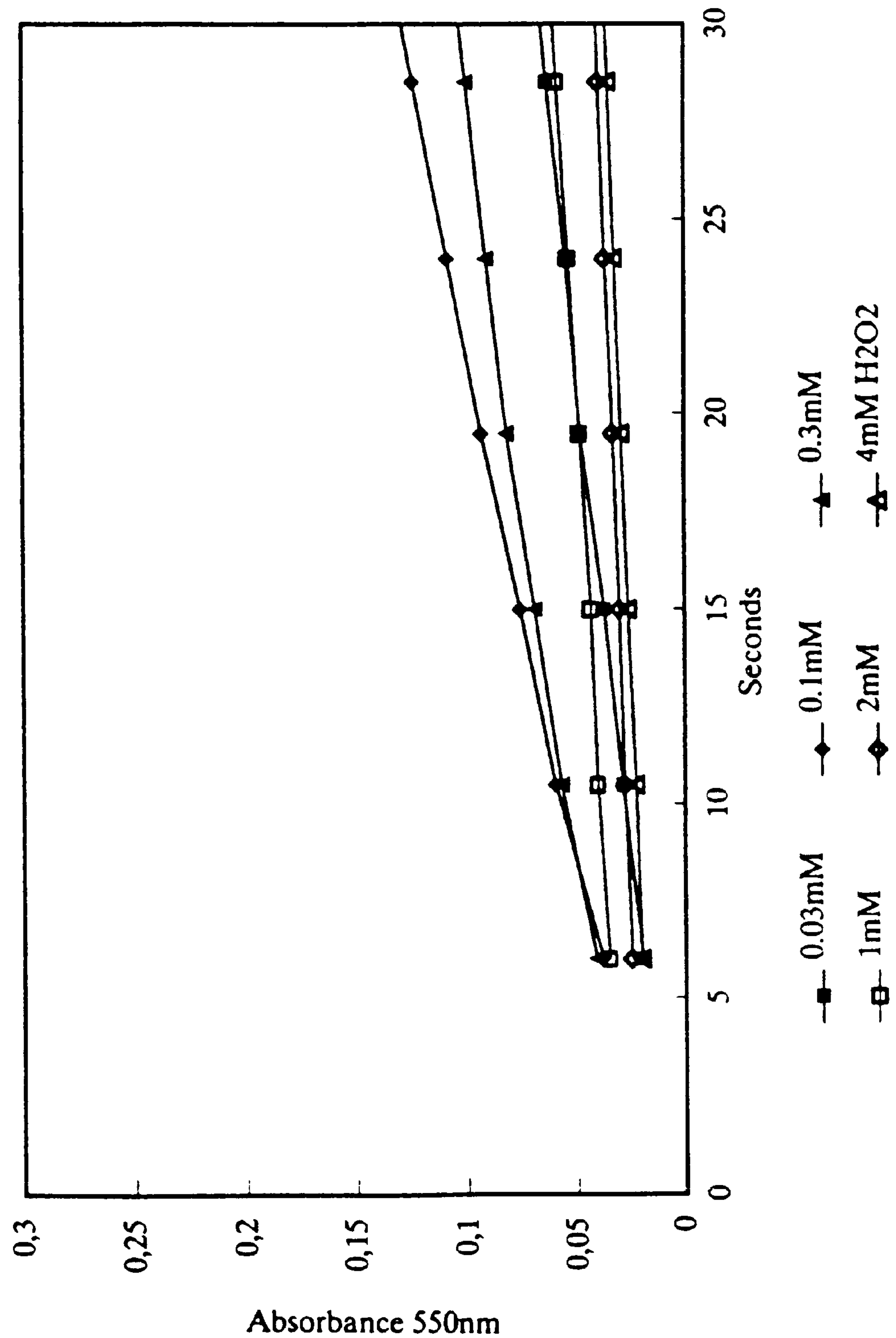
Absorbance 550nm
0,3
0,25
0,2
0,15
0,1
0,05
0
Seconds
0
5
10
15
20
25
30
0.03mM
0.1mM
0.3mM
1mM
2mM
4mM H2O2

Fig. 7

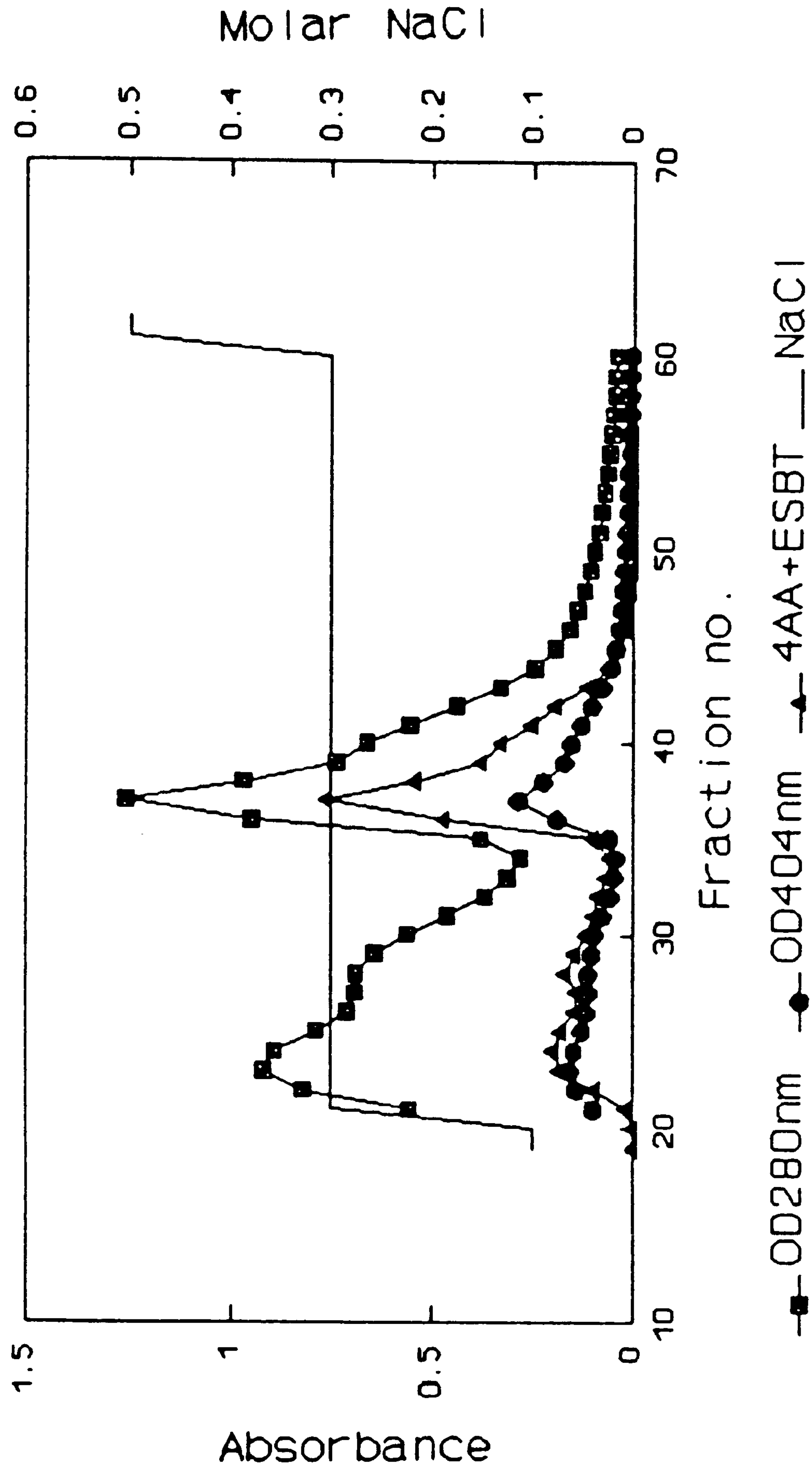
Molar NaCl
0.6
0.5
0.4
0.3
0.2
0.1
0
1.5
1
0.5
0
Absorbance
10
20
30
40
50
60
70
Fraction no.
OD280nm
OD404nm
4AA+ESBT
NaCl

Fig. 8

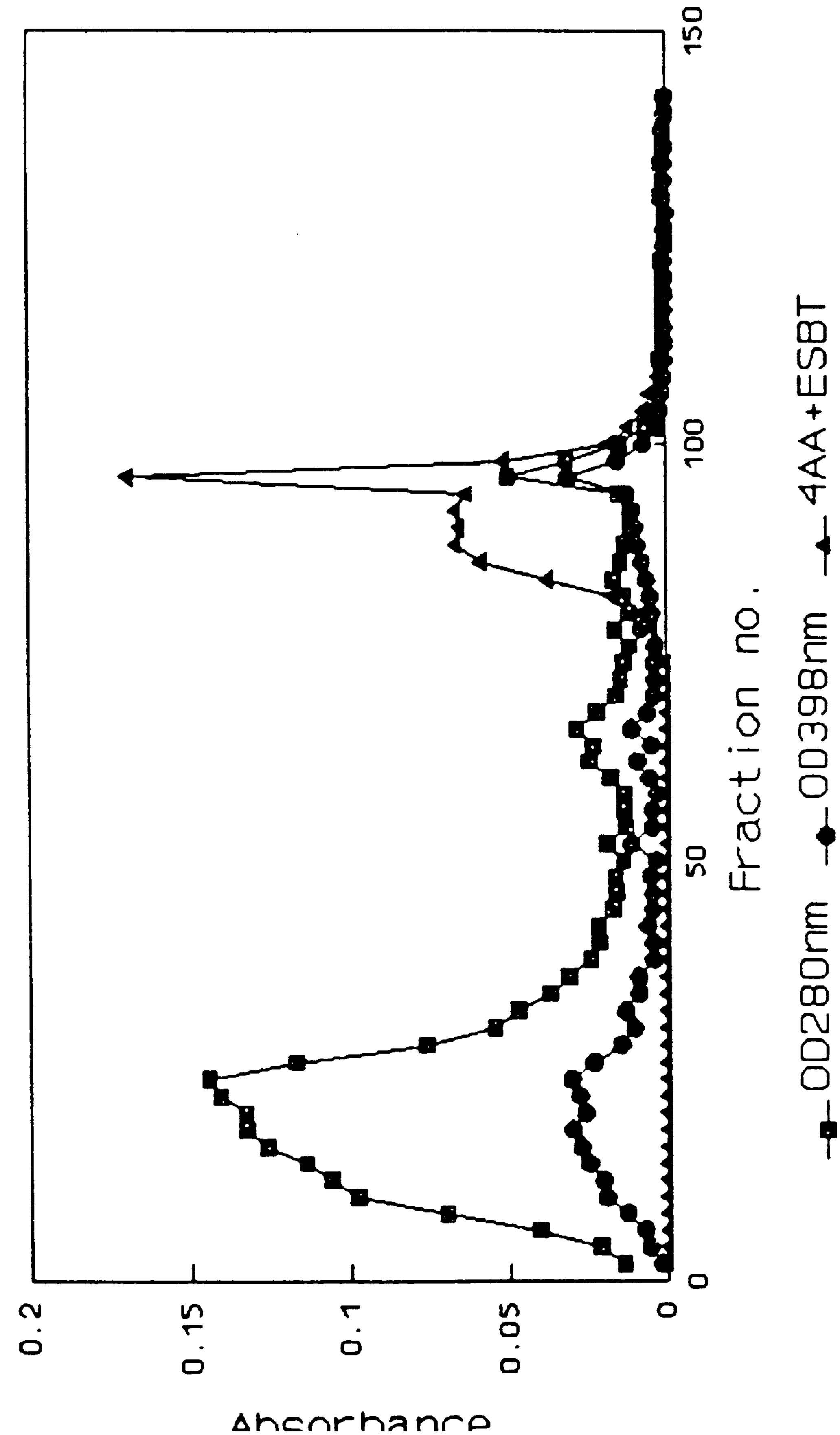
Absorbance
0.2
0.15
0.1
0.05
0
0
50
100
150
Fraction no.
OD280nm
OD398nm
4AA+ESBT

Fig. 9

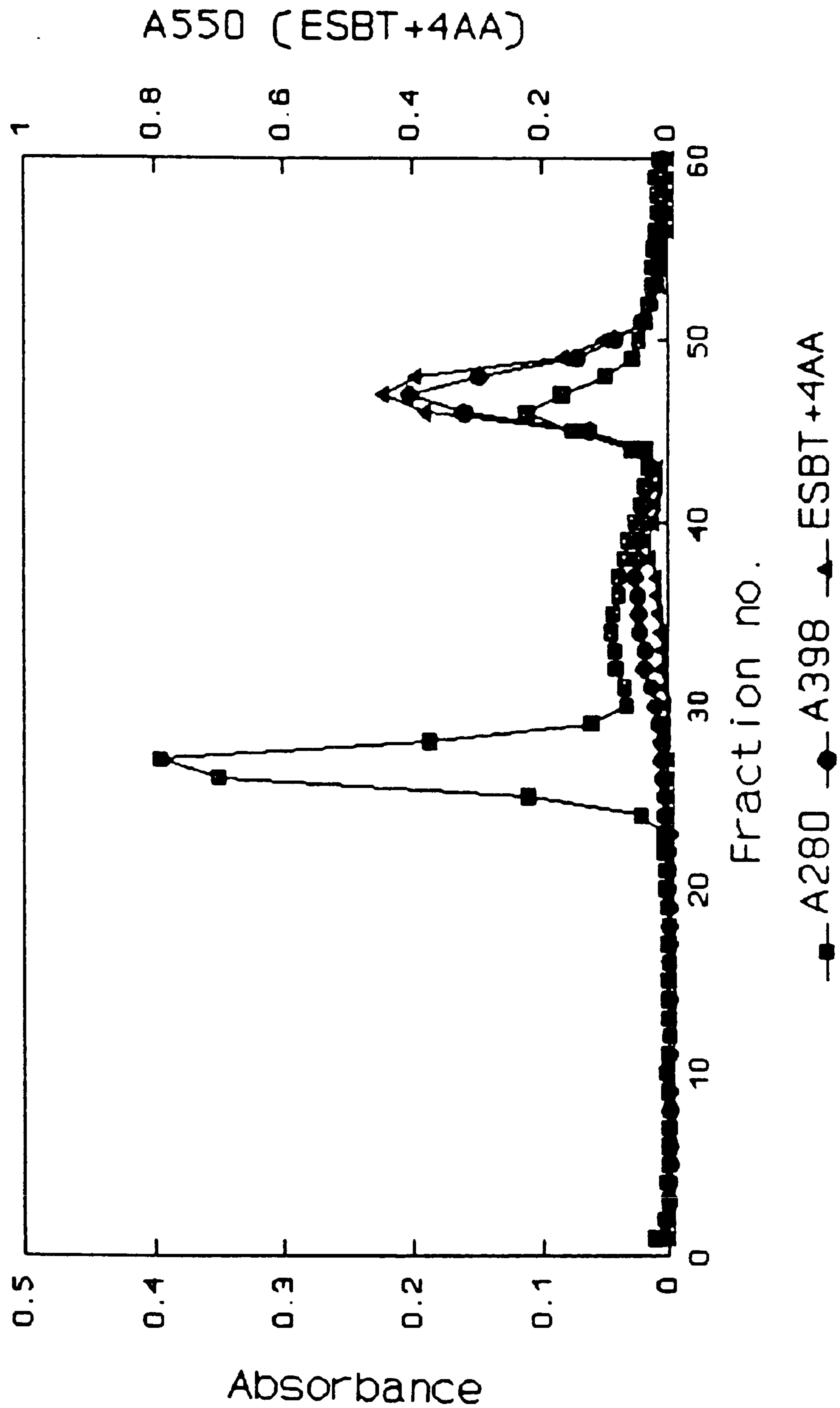
A550 (ESBT+4AA)
1
0.8
0.6
0.4
0.2
0
0.5
0.4
0.3
0.2
0.1
0
Absorbance
0
10
20
30
40
50
60
Fraction no.
A280
A398
ESBT+4AA

Fig. 10

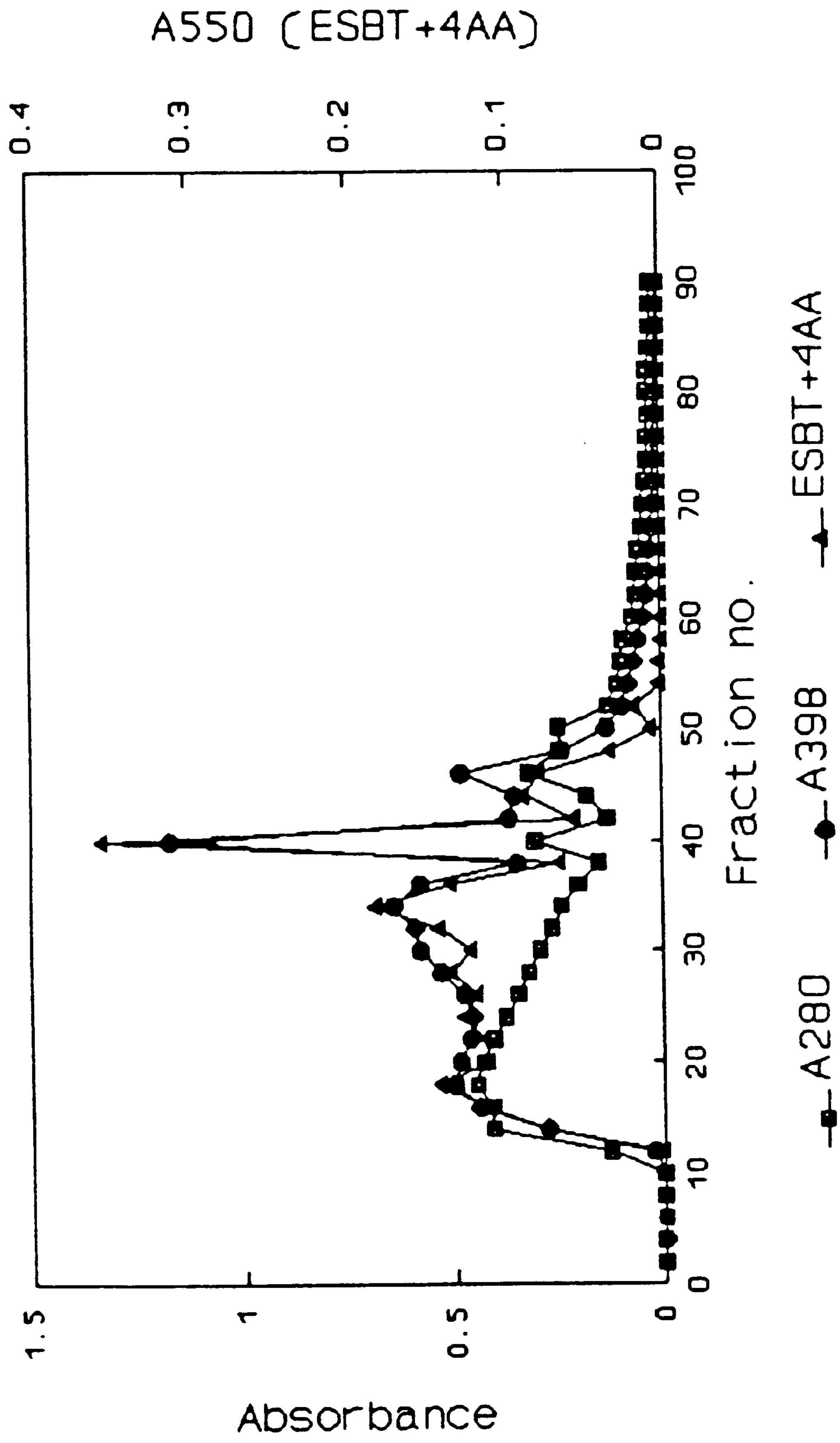
A550 (ESBT+4AA)
0.4
0.3
0.2
0.1
0
1.5
1
0.5
0
Absorbance
0
10
20
30
40
50
60
70
80
90
100
Fraction no.
A280
A398
ESBT+4AA

Fig. 11